Europäisches Patentamt

European Patent Office

Office européen des brevets

(11) Numéro de publication: **0 347 515**

**A1**

## (12) DEMANDE DE BREVET EUROPEEN

(21) Numéro de dépôt: 88870110.9

(22) Date de dépôt: 21.06.88

(51) Int. Cl.4: **A61B 5/08** , **G06F 15/42**

(43) Date de publication de la demande:
27.12.89 Bulletin 89/52

(84) Etats contractants désignés:
**AT BE CH DE ES FR GB GR IT LI LU NL SE**

(71) Demandeur: **BELGIAN ELECTRONIC RESEARCH "B.E.R.", SOCIETE ANONYME**
**Rue des Quatre Grands 13**
**B-4220 Seraing (Jemeppe)(BE)**

(72) Inventeur: **Postiaux, Guy**
**Rue de Miaucourt 43**
**B-6180 Courcelles(BE)**
Inventeur: **Lens, Emile**
**Rue d'Hubes 12**
**B-6180 Courcelles(BE)**

(74) Mandataire: **Vanderperre, Robert et al**
**Bureau VANDER HAEGHEN 63 Avenue de la Toison d'Or**
**B-1060 Bruxelles(BE)** .

(54) **Dispositif d'analyse des bruits respiratoires.**

(57) Un capteur (1) transforme les bruits respiratoires en signaux électriques qui sont reçus dans un circuit échantillonneur/bloqueur (9) pour les échantillonner et produire des échantillons des signaux de mesure, chaque échantillon ayant un niveau constant sur un intervalle de temps prédéterminé. Un convertisseur analogique/numérique (11) convertit chaque échantillon de signal en un signal binaire pour être appliqué à un processeur numérique rapide (12) organisé pour produire un signal représentant le spectre de chaque échantillon du signal de mesure et un micro-ordinateur (14) est organisé pour effectuer sur le signal représentant le spectre de chaque échantillon, un processus d'analyse à la fois dans le domaine fréquentiel et dans le domaine temporel. Le résultat de ces analyses est affiché en temps réel sur un écran de visualisation (16) qui fournit ainsi un monitorage en temps réel des phénomènes respiratoires d'un patient.

Fig. 1

## Dispositif d'analyse des bruits respiratoires

La présente invention concerne un dispositif destiné à l'analyse automatique et continue des bruits respiratoires en temps réel de manière à permettre un monitorage de la respiration en temps réel.

L'auscultation des bruits pulmonaires au moyen d'un stéthoscope reste très subjective car elle dépend beaucoup de l'audition et de l'expérience du médecin.

Certes, d'autres moyens d'investigation existent : radiographie, scanner, scintigraphie, résonance magnétique nucléaire, etc, mais ces techniques sont relativement coûteuses, lourdes d'utilisation et parfois nocives, de toutes façons non utilisables au lit du malade.

L'état actuel de l'acoustique et des techniques d'analyse des signaux permettent d'obtenir des informations plus objectives que par la simple auscultation stéthacoustique. Bien que des équipes de chercheurs se soient intéressés ces dernières années à l'auscultation pulmonaire objective au moyen de techniques modernes, jusqu'à présent ces recherches n'ont cependant pas encore donné le jour à des développements pratiques d'appareils d'utilité diagnostique qui permettraient d'améliorer le diagnostic clinique et la guidance en kinésithérapie respiratoire.

L'invention a pour but de procurer un outil de diagnostic basé sur la technique d'analyse spectrale et temporelle des bruits respiratoires afin de permettre l'analyse automatique et continue des bruits respiratoires en temps réel.

Ce but est atteint, grâce à l'invention, par un dispositif comprenant un capteur pour transformer les bruits respiratoires en signaux électriques, un circuit échantillonneur/bloqueur pour échantillonner lesdits signaux électriques, préalablement amplifiés et filtrés, et produire des échantillons des signaux de mesure, chaque échantillon ayant un niveau constant sur un intervalle de temps prédéterminé, un convertisseur analogique/numérique pour convertir chaque échantillon de signal en un signal binaire pour être appliqué à un processeur numérique rapide organisé pour produire un signal représentant le spectre de chaque échantillon du signal de mesure et un micro-ordinateur effectue sur le signal représentant le spectre de chaque échantillon, un processus d'analyse à la fois dans le domaine fréquentiel et dans le domaine temporel, et pour afficher en temps réel sur un écran de visualisation, un histogramme de la fréquence de sibilance et le signal spectral pendant une période de temps prédéterminée.

Le micro-ordinateur et le processeur numérique rapide sont organisés pour effectuer un processus d'analyse sur le signal représentant le spectre de chaque échantillon et sur le signal temporel, appelé sonospirogramme.

Le processus d'analyse effectué sur le signal représentant le spectre de chaque échantillon se caractérise par les étapes suivantes :
- acquisition de chaque signal;
- commande de l'affichage de chaque signal pendant un intervalle de temps prédéterminé:
- détermination de l'amplitude maximum du spectre et de la fréquence dominante du spectre;
- détermination de l'amplitude moyenne du spectre dans la bande de fréquences 200 Hz - 1 kHz;
- multiplication de ladite amplitude moyenne avec un coefficient mémorisé, représentant un facteur de consigne prédéterminé;
- comparaison de l'amplitude maximum Amax avec la valeur KSmoy et production d'un signal WH ayant deux états : un état 0 lorsque l'amplitude maximum Amax est inférieure à la valeur KSmoy maximum et un état 1 lorsque l'amplitude maximum Amax est supérieure à la valeur KSmoy;
- lorsque ledit signal WH est à l'état 1, incrémentation d'un compteur de sibilance et enregistrement de la fréquence correspondant à l'amplitude maximum Amax du spectre (fréquence de sibilance);
- affichage de la fréquence de sibilance pour chaque échantillon pendant un intervalle de temps prédéterminé;
- calcul du rapport de la durée du signal WH à l'état 1 (état de sibilance) à une période de temps prédéterminée (par exemple 20 secondes) et production et mémorisation, pour chaque période, d'un signal WH% représentant le pourcentage de durée de sibilance par période;
- commande de l'affichage des taux de sibilance WH% sur l'écran de visualisation.

Le processus d'analyse effectué sur le signal St représentant la courbe sonospirogramme se caractérise par les étapes suivantes :
- détermination du maximum Max1 de la forme d'onde dans une première fenêtre temporelle T1 et production d'un signal à l'instant t1 correspondant audit maximum;
- à partir de l'instant t1, détermination du minimum Min1 de la forme d'onde dans une deuxième fenêtre temporelle T2 et production d'un signal à l'instant t2 correspondant audit minimum Min1;
- à partir de l'instant t2, détermination du maximum Max2 de la forme d'onde dans une troisième fenêtre temporelle T3 et production d'un signal à l'instant t3 correspondant audit maximum Max2;
- à partir de l'instant t3, détermination du minimum Min2 de la forme d'onde dans la fenêtre temporelle T2 et production d'un signal à l'instant t4 correspondant audit minimum Min2;

- à partir de l'instant t4, détermination du maximum Max3 de la forme d'onde dans la fenêtre temporelle T3 et production d'un signal à l'instant t5 correspondant audit maximum Max3;

- à partir de l'instant t5, détermination du minimum Min3 de la forme d'onde dans la fenêtre temporelle T2 et production d'un signal à l'instant t6 correspondant audit minimum Min3;

- calcul des valeurs I/E et RR et mise en mémoire de ces valeurs; les valeurs I/E et RR étant déterminées d'après les équations suivantes :

$$I/E = (t_{Min2} - t_{Min1})/(t_{Min3} - t_{Min2}) \text{ et}$$
$$RR = 1/(I + E) = 1/(t_{Min3} - t_{Min1});$$

- répétition des étapes précédentes;

- après un laps de temps prédéterminé (par exemple 20 secondes), calcul des valeurs moyennes des signaux I/E et RR et commande de l'affichage de ces valeurs moyennes.

D'autres particularités du dispositif apparaîtront à la lecture de la description qui suit, faite en s'appuyant sur les dessins ci-annexés dans lequels :

- La figure 1 est un schéma par blocs d'un mode d'exécution exemplaire du dispositif selon l'invention.

- Les figures 2 et 3 sont des organigrammes illustrant le processus de traitement des signaux de mesure selon l'invention.

- Les figures 4 et 5 sont des diagrammes montrant un spectre fréquentiel exemplaire et un signal temporel exemplaire appelé sonospirogramme.

- La figure 6 montre un exemple de graphiques présentés sur l'écran de visualisation du dispositif selon l'invention.

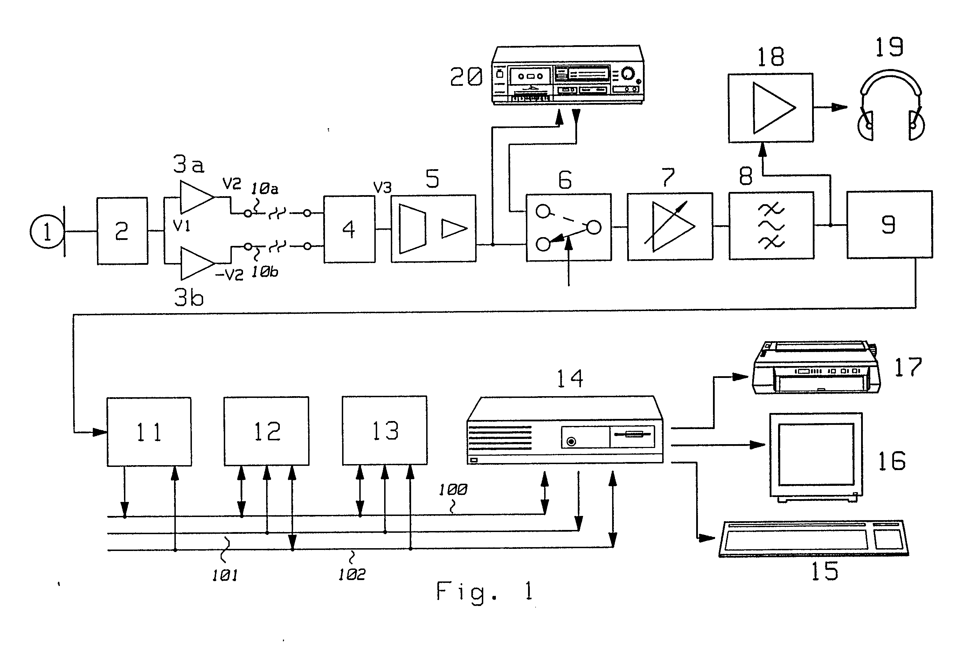

Se reportant à la figure 1, on voit un capteur 1 servant à capter les bruits respiratoires d'un patient et les transformer en signaux électriques. Le capteur est constitué d'un microphone à condensateur logé dans un boîtier en polyéthylène muni d'une collerette adhésive jetable destinée à fixer le boîtier sur le creux sternal du patient. Le boîtier est réalisé de façon à isoler le microphone du bruit ambiant. Le microphone est évidemment choisi pour avoir une bande passante adéquate 10 Hz à 20 kHz. Les signaux électriques produits par le microphone sont reçus dans un circuit d'entrée 2 comprenant un étage suiveur et un préamplificateur, puis ils sont appliqués à un circuit de symétrisation 3 ayant pour but d'éliminer les signaux parasites dans les signaux audiofréquences utiles. Le circuit de symétrisation comprend deux branches : la première branche contient un amplificateur 3a qui délivre dans une première voie de transmission 10a un signal amplifié V2, la seconde branche contient un amplificateur à inversion 3b qui délivre dans une voie de transmission 10b un signal amplifié inversé -V2. Les circuits d'entrée et de symétrisation sont logés dans un boîtier associé au microphone.

Les voies de transmission 10a et 10b peuvent être réalisées sous diverses formes techniques connues : conducteurs électriques, liaisons hertziennes, faisceaux infrarouges, fibres optiques ou autres. Dans la figure 1 on les a représentées de façon simplifiée par deux conducteurs d'un câble de liaison 10 qui relie le boîtier associé au microphone et le dispositif d'analyse fixe.

A son extrémité terminale le câble de liaison 10 est connecté à un circuit soustracteur 4 agencé pour soustraire l'un de l'autre les signaux V2 et -V2 reçus des deux voies de transmission 10a et 10b et pour produire un signal de différence V3 qui est appliqué au dispositif d'analyse. Les signaux parasites éventuels qui se superposent au signal utile dans les voies de transmission 10a et 10b se trouvent éliminés dans le soustracteur 4 qui délivre ainsi un signal utile V3 débarrassé de tout parasite. Un amplificateur d'isolation 5 assure ensuite une isolation galvanique entre le dispositif appliqué au patient et l'équipement d'analyse fixe.

La sortie de l'amplificateur d'isolation 5 est connectée à un amplificateur opérationnel à gain réglable 7 suivi d'une cellule de filtration 8 comprenant un filtre passe-bande et un réjecteur de 50 Hz pour éliminer l'influence du secteur. Le filtre passe-bande est par exemple un filtre de Butterworth du 8e ordre.

Le signal utile, amplifié et filtré, est appliqué à l'entrée d'un circuit échantillonneur et bloqueur 9 agencé pour échantillonner le signal utile à une fréquence d'échantillonnage prédéterminée, par exemple une fréquence de 2048 Hz, et pour maintenir le niveau de chaque échantillon constant pendant la durée de la mesure.

Dans un mode d'exécution, les circuits constituant les blocs 4 à 9 et le bloc 18 dans la figure 1 sont réunis dans un boîtier. Celui-ci comprend avantageusement un commutateur 6 pour permettre à l'utilisateur de sélectionner soit un monitorage direct en temps réel à partir du capteur, soit une mesure indirecte à partir d'un enregistrement de bruits respiratoires sur cassette 20. L'entrée de l'échantillonneur 9 est également avantageusement connectée à un amplificateur 18 pour le raccordement d'écouteurs 19, permettant le suivi auditif des tracés présentés sur l'écran 16. Dans un autre mode d'exécution les circuits, des blocs 4 à 9 et 18 peuvent être incorporés dans l'ensemble d'analyse avec les circuits 11 à 13 qui sont décrits ci-après.

Les échantillons du signal de mesure sont reçus dans un convertisseur analogique/numérique 11 pour convertir le signal de mesure analogique en signal numérique. Le convertisseur a une résolution suffisante, par exemple huit à seize bits, et effectue la conversion par approximations successives.

Les signaux de mesure numériques fournis par le convertisseur 11 sont reçus dans un ensemble de traitement de données auquel le convertisseur est relié par un bus de données 100, un bus d'adresse 101 et un bus de commande 102 ainsi qu'il est usuel dans le domaine de l'art. L'ensemble de traitement de données comprend essentiellement un processeur numérique rapide 12, une mémoire 13 et un micro-ordinateur 14 auquel sont associés un clavier de commande 15, un écran de visualisation 16 et une imprimante 17. Bien que représentée séparément dans la figure 1, la mémoire 13 peut parfaitement être une zone réservée dans la ou une mémoire incorporée dans le micro-ordinateur. Dans un mode de réalisation exemplaire, le convertisseur 11, le processeur rapide 12 et la mémoire 13 sont réunis sur une carte imprimée logée dans le micro-ordinateur 14. Il est bien entendu cependant que l'ensemble du dispositif d'analyse pourrait également être réuni dans un appareil intégré portable.

Le processeur numérique rapide 12 et le microordinateur 14 sont organisés pour effectuer une transformation de Fourier rapide, connue en soi, afin de produire la transformée discrète FFT donnant le spectre du signal de mesure. Le spectre est calculé toutes les 250 millisecondes par exemple, ce qui correspond à 512 échantillons numériques du signal de mesure. Les signaux S représentant le spectre de chaque échantillon sont mémorisés dans la mémoire 13. Le micro-ordinateur 14 effectue sur les signaux S de chaque spectre d'échantillon un processus de traitement spécifique en temps réel dans le domaine fréquentiel. Toutes les étapes du processus de traitement sont exécutées en réponse à des signaux de commande mémorisés sur disquette par exemple. Les échanges de signaux de commande entre le processeur rapide 12, la mémoire 13 et le microprocesseur 14 se font par le bus de commande 102; les échanges de signaux d'adresse se font par l'intermédiaire du bus d'adresse 101.

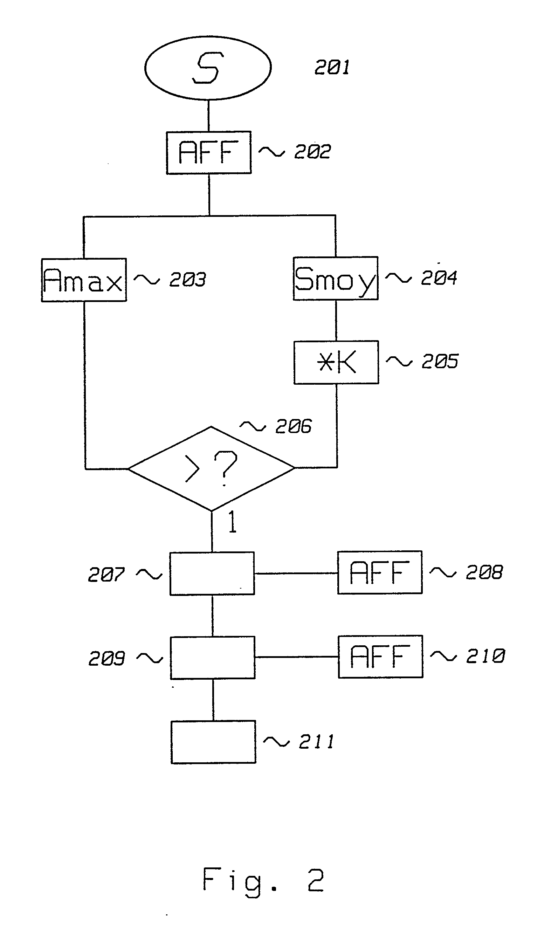

Le processeur numérique rapide 12 et le microordinateur 14 sont organisés pour répondre aux signaux de commande de manière à exécuter un processus d'analyse dans le domaine fréquentiel sur des périodes de temps de trente secondes. Ce processus est illustré par l'organigramme de la figure 2. Les étapes fonctionnelles significatives de ce processus sont décrites ci-après.

- Etape 201 : acquisition des signaux S.

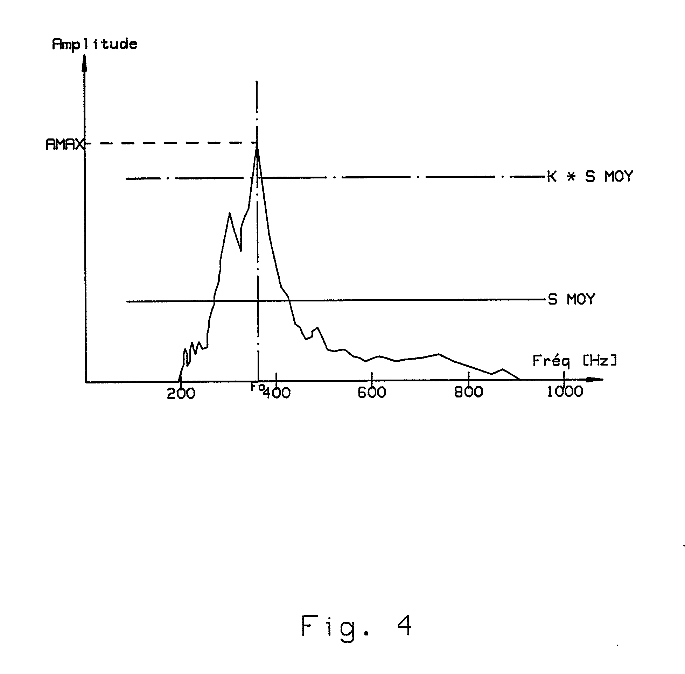

La figure 4 illustre un exemple de signal de spectre d'échantillon.

- Etape 202 : commande de l'affichage des signaux S reçus pendant un intervalle de trente secondes, par exemple.
- Etape 203 : détermination de l'amplitude maximum du spectre Amax de chaque échantillon et de la fréqence dominante Fo du spectre.
- Etape 204 : détermination de l'amplitude moyenne du spectre de chaque échantillon (Smoy) dans la bande de fréquences utile 200 Hz - 1 kHz.
- Etape 205 : multiplication de l'amplitude moyenne Smoy avec un coefficient K mémorisé, représentant un facteur de consigne prédéterminé, et production et mémorisation d'un signal représentant la valeur KSmoy.
- Etape 206 : comparaison de l'amplitude maximum Amax avec la valeur KSmoy et production d'un signal WH ayant deux états : un état 0 lorsque l'amplitude maximum Amax est inférieure à la valeur KSmoy (absence de sibilance) et un état 1 lorsque l'amplitude maximum Amax est supérieure à la valeur KSmoy (état de sibilance).
- Etape 207 : lorsque le signal WH est à l'état 1, incrémentation d'un compteur de sibilance et enregistrement de la fréquence de sibilance (fréquence Fo correspondant à l'amplitude maximum du spectre (étape 203).
- Etape 208 : commande de l'affichage de la fréquence de sibilance pour chaque échantillon pendant un intervalle de trente secondes par exemple.
- Etape 209 : calcul du rapport de la durée du signal WH à l'état 1 (état de sibilance) à une période de temps de 20 secondes par exemple et production, pour chaque période, d'un signal WH% représentant le pourcentage de durée de sibilance par période.
- Etape 210 : commande de l'affichage des taux de sibilance WH%.
- Etape 211 : mise en mémoire des signaux WH et WH% pour rappel ultérieur.

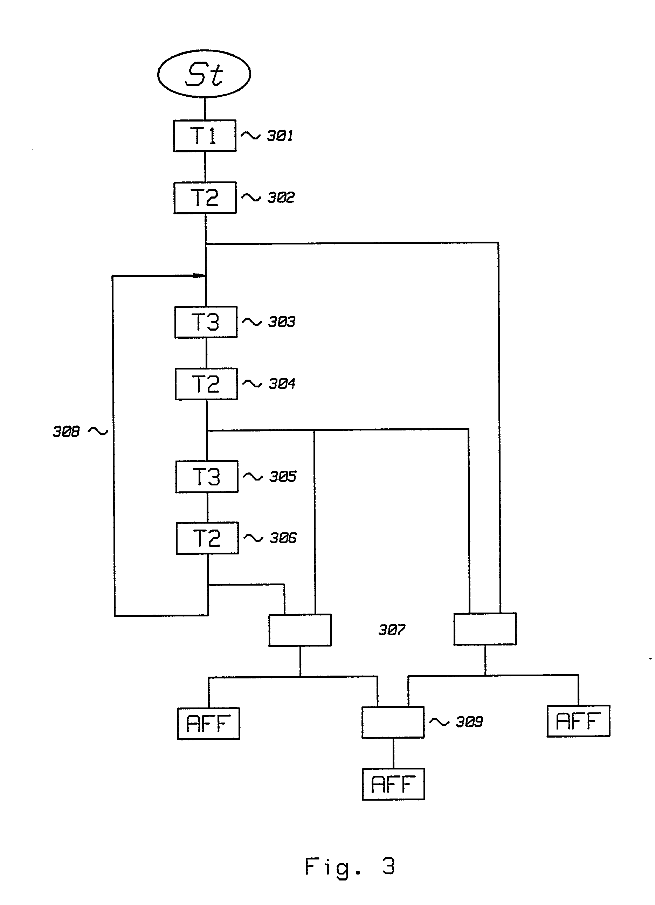

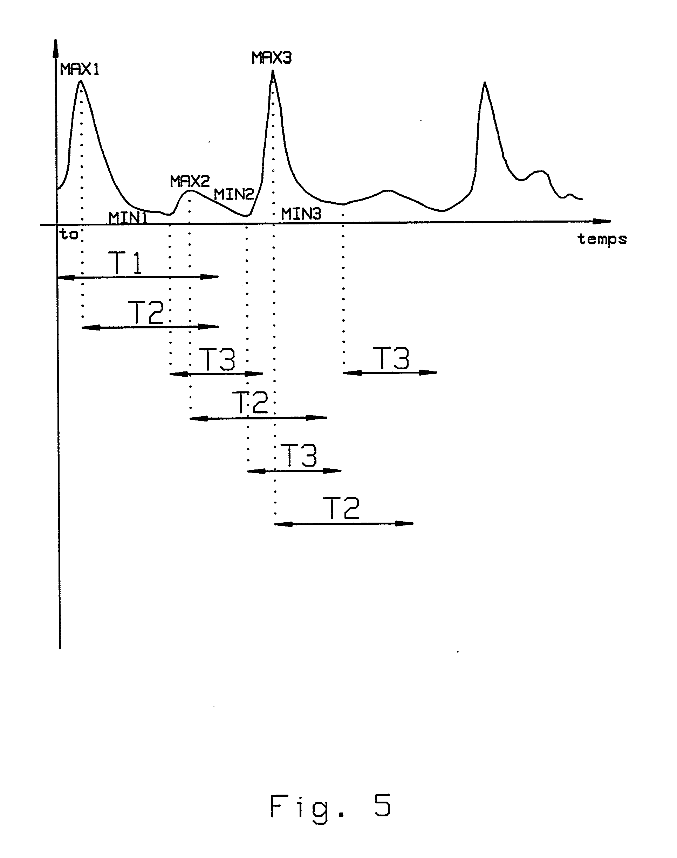

Parallèlement au processus décrit ci-dessus, le processeur numérique rapide 12 et le micro-ordinateur 14 déterminent aussi dans le domaine temporel la valeur moyenne des valeurs absolues des 512 échantillons, l'ensemble des valeurs moyennes formant la courbe sonospirogramme St. Un exemple de courbe sonospirogramme St est montré à la figure 5. En vue d'extraire la valeur RR représentant le rythme respiratoire et le rapport I/E de la phase d'Inspiration sur la phase d'Expiration, le processeur numérique rapide 12 exécute sur la forme d'onde du signal St, un processus d'analyse sur des périodes de temps prédéterminées, par exemple trente secondes. Ce processus est illustré par l'organigramme de la figure 3.

- Etape 301 : détermination du maximum Max1 de la forme d'onde dans une fenêtre temporelle T1 et production d'un signal à l'instant t1 correspondant audit maximum.

- Etape 302 : à partir de l'instant t1, détermination du minimum Min1 de la forme d'onde dans une fenêtre temporelle T2 et production d'un signal à l'instant t2 correspondant au minimum et d'un signal Min1 représentant ledit minimum.

- Etape 303 : à partir de l'instant t2, détermination du maximum Max2 de la forme d'onde dans une fenêtre temporelle T3 et production d'un signal à l'instant t3 correspondant au maximum.

- Etape 304 : à partir de l'instant t3, détermination du minimum Min2 de la forme d'onde dans une fenêtre temporelle T2 et production d'un signal à l'instant t4 correspondant au minimum et d'un signal Min2 représentant ledit minimum.

- Etape 305 : à partir de l'instant t4, détermination du maximum Max3 de la forme d'onde dans une fenêtre temporelle T3 et production d'un signal à l'instant t5 correspondant au maximum.

- Etape 306 : à partir de l'instant t5, détermination du minimum Min3 de la forme d'onde dans une fenêtre temporelle T2 et production d'un signal à l'instant t6 correspondant au minimum et d'un signal Min3 représentant ledit minimum.

- Etape 307 : calcul des valeurs I/E et RR et mise en mémoire de ces valeurs; les valeurs I/E et RR sont déterminées à partir des signaux Min1, Min2 et Min3 d'après les équations suivantes :

$$I/E = (t_{Min2} - t_{Min1})/(t_{Min3} - t_{Min2})$$ et
$$RR = 1/(I + E) = 1/(t_{Min3} - t_{Min1}).$$

- Etape 308 : répétition des étapes 303 à 307.

- Etape 309 : après 20 secondes, par exemple, calcul des valeurs moyennes des signaux I/E et RR, production de signaux représentant ces valeurs et affichage de ces signaux représentant les valeurs moyennes.

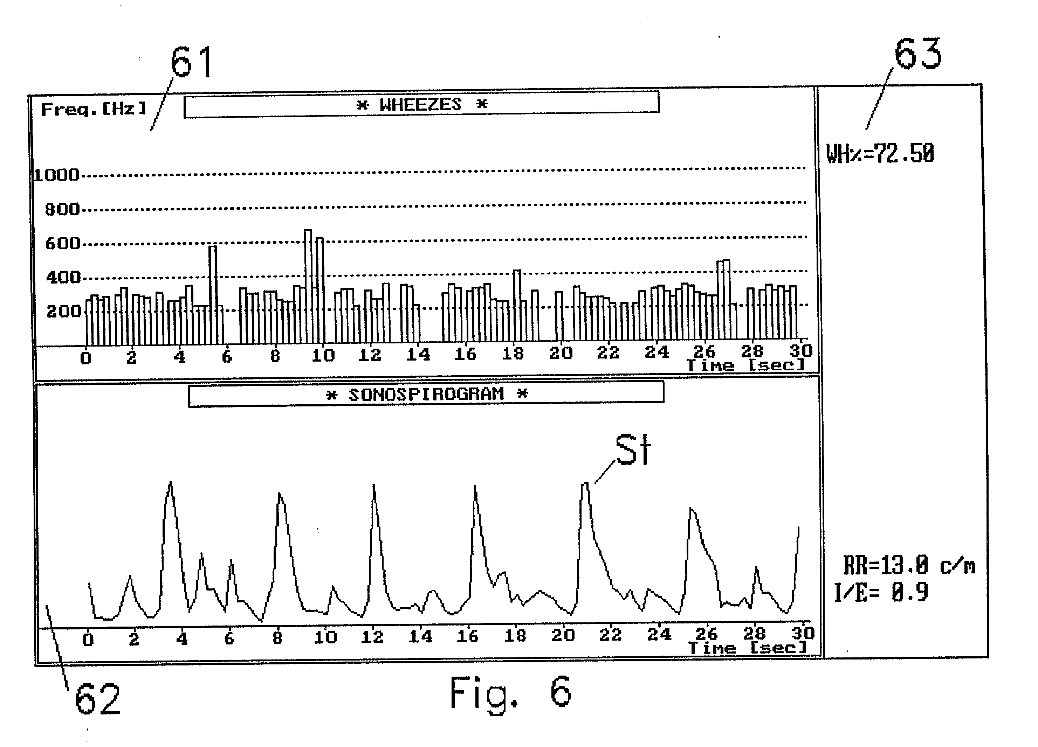

Le dispositif d'analyse selon l'invention détermine ainsi automatiquement et simultanément plusieurs paramètres physiologiques et pathologiques par monitorage en temps réel du seul bruit respiratoire et apporte ainsi au médecin un outil de diagnostic lui permettant un monitorage efficace de l'asthme et des troubles respiratoires en temps réel. La figure 6 montre un exemple d'affichage sur écran de visualisation particulièrement original et utile. La surface de visualisation est divisée en trois zones : la zone 61 présente un histogramme de la fréquence de sibilance d'un patient en fonction du temps (produit au cours de l'étape 207 décrite plus haut), la zone 62 présente la courbe sonospirogramme (courbe St) sur une échelle de temps correspondante, et la zone 63 sert à l'affichage des valeurs WH% (taux de sibilance), RR (rythme respiratoire du patient) et I/E (rapport de la phase d'inspiration à la phase d'expiration) produites en temps réel par le micro-ordinateur. Le dispositif peut être prévu pour permettre de superposer le sonospirogramme sur l'histogramme. Grâce au dispositif selon l'inven tion, le praticien bénéficie donc d'un monitorage en temps réel particulièrement efficace qui lui permet d'emblée d'avoir une vue globale sur le phénomène respiratoire d'un patient et de faire rapidement un diagnostic précis. Le dispositif peut également assurer la surveillance simultanée de plusieurs patients grâce à plusieurs capteurs raccordés au dispositif d'analyse par liaison avec ou sans fil.

L'imprimante qui complète le dispositif d'analyse permet de fournir des synoptiques et rapports graphiques et/ou numériques qui donnent de façon avantageuse des enregistrements permanents utiles sur des périodes de temps continues longues, par exemple des périodes de vingt-quatre heures.

Bien que dans ce qui précède on ait décrit une forme de réalisation avantageuse de l'invention à titre d'exemple, il est entendu que l'invention n'est nullement limitée à celle-ci. Toutes les modifications et variantes ou tous agencements équivalents qui sont de la compétence de l'homme de l'art doivent être considérés comme compris dans le cadre de l'invention.

Ainsi, par exemple, le circuit échantillonneur/bloqueur, le convertisseur analogique/numérique, le processeur numérique rapide et sa mémoire pourraient être remplacés par un analyseur de Fourier classique. De même, le processeur numérique rapide ou un analyseur de Fourier pourraient être omis dans le cas où le processeur du micro-ordinateur ou de tout autre ordinateur aurait une puissance de calcul suffisante.

## Revendications

1. Dispositif pour l'analyse automatique des bruits respiratoires en temps réel, comprenant un capteur pour transformer les bruits respiratoires en signaux électriques, un circuit échantillonneur/bloqueur (9) pour échantillonner les signaux de bruits respiratoires, préalablement amplifiés et filtrés, et produire des échantillons des signaux de mesure, chaque échantillon ayant un niveau constant sur un intervalle de temps prédéterminé, un convertisseur analogique/numérique (11) pour convertir chaque échantillon de signal en un signal binaire pour être appliqué à un processeur numérique rapide (12) organisé pour produire un signal représentant le spectre de chaque échantillon du signal de mesure et un micro-ordinateur (14) organisé pour effectuer sur la sortie du processeur numérique rapide (12) un processus d'analyse spectrale et temporelle, et pour afficher en temps réel sur un écran de visualisation (16) un histogramme de la fréquence de sibilance (Fo) et le signal spectral (St) pendant une période de temps prédéterminée.

2. Dispositif selon la revendication 1, caractérisé en ce que le processus d'analyse effectué sur le signal (S) représentant le spectre de chaque échantillon comprend les étapes suivantes :
- acquisition de chaque signal (S);
- commande de l'affichage de chaque signal (S) pendant un intervalle de temps prédéterminé;
- détermination de l'amplitude maximum du spectre (Amax) et de la fréquence dominante du spectre (Fo);
- détermination de l'amplitude moyenne du spectre (Smoy) dans la bande de fréquences 200 Hz - 1 kHz;
- multiplication de ladite amplitude moyenne (Smoy) avec un coefficient mémorisé (K), représentant un facteur de consigne prédéterminé;
- comparaison de l'amplitude maximum (Amax) avec la valeur (KSmoy) et production d'un signal (WH) ayant deux états : un état 0 lorsque l'amplitude maximum (Amax) est inférieure à la valeur (KSmoy) et un état 1 lorsque l'amplitude maximum (Amax) est supérieure à la valeur (KSmoy);
- lorsque ledit signal (WH) est à l'état 1, incrémentation d'un compteur de sibilance et enregistrement de la fréquence correspondant à l'amplitude maximum (Amax) du spectre (fréquence de sibilance);
- affichage de la fréquence de sibilance pour chaque échantillon pendant un intervalle de temps prédéterminé;
- calcul du rapport de la durée du signal (WH) à l'état 1 (état de sibilance) à une période de temps prédéterminée (par exemple 20 secondes) et production, pour chaque période, d'un signal (WH%) représentant le pourcentage de durée de sibilance par période;
- commande de l'affichage des taux de sibilance (WH%).

3. Dispositif selon la revendication 1 ou 2, caractérisé en ce que le processus d'analyse effectué sur le signal (St) représentant la courbe sonospirogramme comprend les étapes suivantes :
- détermination du maximum (Max1) de la forme d'onde dans une première fenêtre temporelle (T1) et production d'un signal à l'instant (t1) correspondant audit maximum:
- à partir de l'instant t1, détermination du minimum (Min1) de la forme d'onde dans une deuxième fenêtre temporelle (T2) et production d'un signal à l'instant (t2) correspondant audit minimum (Min1);
- à partir de l'instant t2, détermination du maximum (Max2) de la forme d'onde dans une troisième fenêtre temporelle (T3) et production d'un signal à l'instant (t3) correspondant audit maximum (Max2);
- à partir de l'instant t3, détermination du minimum (Min2) de la forme d'onde dans la fenêtre temporelle (T2) et production d'un signal à l'instant (t4) correspondant audit minimum (Min2);
- à partir de l'instant t4, détermination du maximum (Max3) dé la forme d'onde dans la fenêtre temporelle (T3) et production d'un signal à l'instant (t5) correspondant audit maximum (Max3);
- à partir de l'instant t5, détermination du minimum (Min3) de la forme d'onde dans la fenêtre temporelle (T2) et production d'un signal à l'instant (t6) correspondant audit minimum (Min3);
- calcul des valeurs I/E et RR et mise en mémoire de ces valeurs; les valeurs I/E et RR étant déterminées d'après les équations suivantes :
$I/E = (t_{Min2} - t_{Min1})/(t_{Min3} - t_{Min2})$ et
$RR = 1/(I + E) = 1/(t_{Min3} - t_{Min1})$;
- répétition des étapes précédentes;
- après un laps de temps prédéterminé (par exemple 20 secondes), calcul des valeurs moyennes des signaux I/E et RR et commande de l'affichage de ces valeurs moyennes.

4. Dispositif selon l'une quelconque des revendications précédentes, caractérisé en ce qu'il comprend en outre un circuit connecté entre le capteur (1) et le dispositif d'analyse fixe, ce circuit comprenant deux branches connectées pour recevoir le signal du capteur, la première branche contenant un amplificateur (3a) pour produire un signal amplifié (V2) dans une première voie de transmission (10a) et la seconde branche contenant un amplificateur à inversion (3b) pour produire un signal amplifié inversé (-V2) dans une seconde voie de transmission (10b), et un circuit soustracteur (4) pour soustraire l'un de l'autre les signaux (V2 et -V2) produits par les deux amplificateurs (3a, 3b) précités et pour produire un signal de différence (V3) pour l'échantillonneur/bloqueur (9).

5. Dispositif selon l'une quelconque des revendications précédentes, caractérisé en ce que le convertisseur analogique/numérique (11) et le processeur rapide (12) sont réunis sur une carte à circuit imprimé montée dans le micro-ordinateur.

6. Dispositif selon la revendication 5, caractérisé en ce que la carte à circuit imprimé porte également une mémoire (13).

Fig. 1

EP 0 347 515 A1

Fig. 2

Fig. 3

Fig. 4

Fig. 5

Fig. 6

EP 0 347 515 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | L'ONDE ELECTRIQUE vol. 67, no. 4-5, Juillet/Septembre 1987, pages 100-104, Paris, F; M. SUDRAUD et al.:"Sons pulmonaires" * Résumé, page 101, colonne de gauche, ligne 25 - page 103, colonne de gauche, ligne 38; figures 1,2 * --- | 1,5,6 | A 61 B  5/08 G 06 F  15/42 |
| A | DE-A-3 044 639  (DR. KARL THOMAE GMBH) * page 1, ligne 1 - page 2, ligne 25; figure 1 et "BLOCKDIGRAM" * --- | 1,5,6 | |
| A | WO-A-8 605 965  (EMERGENT TECHNOLOGY CORPORATION) * page 12, ligne 3 - page 13, ligne 11; page 14, ligne 16 - page 16, ligne 15; figures 1 A, 1 C, 1 D,2 * ----- | 1 | |

|  |  |  | DOMAINES TECHNIQUES RECHERCHES (Int. Cl.4) |
|---|---|---|---|
|  |  |  | A 61 B  5/00 G 06 F  15/00 |

Le présent rapport a été établi pour toutes les revendications

| Lieu de la recherche | Date d'achèvement de la recherche | Examinateur |
|---|---|---|
| BERLIN | 23-02-1989 | WEIHS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&: membre de la même famille, document correspondant

EPO FORM 1503 03.82 (P0402)